# EUROPEAN PATENT APPLICATION

(11) **EP 1 281 407 A1**
(43) Date of publication of application: **05.02.2003**
(21) Application number: 01810746.6
(22) Date of filing: 30.07.2001
(51) Int. Cl.: A61M 1/36

(54) **Method of continuously separating whole blood and device for carrying out this method**

(71) Applicant: Rochat, Jean-Denis, 1272 Genolier (CH)
(72) Inventor: Rochat, Jean-Denis, 1272 Genolier (CH)
(74) Representative: Savoye, Jean-Paul

(57) **Abstract**

According to this method of continuously separating whole blood as well as any liquid containing human cells comprising the steps of: forming a substantially laminar flow of said blood and submitting said flow to a force field directed transversely to the direction of said flow to get the red blood cells and at least the platelets separated, and discharging said separated components out of said flow, the platelet-poor plasma continuously collected as it is formed, along at least a portion of the length of said flow.

## Description

The present invention relates to a method of continuously separating whole blood as well as any liquid containing human cells comprising the steps of: forming a flow of said blood and submitting said flow to a force field directed transversely to the direction of said flow to get the red blood cells and at least the platelets separated, and discharging said separated components out of said flow.

This invention also relates to a blood component separating device which comprises a centrifuge member adapted for rotation about its central axis in a centrifuge, said member having two outer, respectively inner circular walls spaced from each other to define a separating room comprising an inlet opening to be connected to a supply of blood and at least two outlet openings, an outlet opening to be connected to means for collecting a higher density component of blood, another outlet opening to be connected to means for collecting a lower density component, said inlet and other outlet openings being respectively adjacent to two upstream, respectively downstream ends angularly spaced of said separating room.

Conventional blood processing devices and methods use centrifuge circular disposable chambers into which whole blood is introduced while rotating them to create a centrifugal field.

Whole blood submitted to the force of the centrifugal field resulting from the rotation of the chamber, separates into higher density red blood cells (RBC) and lower density platelet-rich plasma (PRP). An intermediate layer of white blood cells and lymphocytes is formed between the red blood cells and platelet-rich plasma.

In order to obtain a good platelet recovery efficency (the ratio between the collected platelets (PLT) and the platelets in the whole blood), it is necessary to take them out from RBC and from plasma. For taking out PLT from RBC, RBC have to be concentrated to a maximum, since the collection of platelets is proportionnal to plasma caught between the RBC. In the known separation chambers, 2/3 to 3/4 of its overall length is used for concentrating RBC to a maximum. The separation under the influence of centrifugal force field results in a concentration of RBC against the outer wall of the circular chamber, whereas the platelets are driven into the platelet-rich plasma which is along the inner wall.

The peripheral length of the circular separating chamber is determined by the sedimentation paths of platelets into the plasma, the platelet sedimentation speed being 15 times slower than that of RBC. Therefore, if it would be possible to shorten the sedimentation paths of platelets, the length of the separating chamber and thus its diameter could also be proportionally reduced.

Indeed, as it will be explained hereunder, if we take into account a platelet path in the worst operating conditions, that is to say that when the platelet is the smallest and when it is released from RBC on the smallest radius of the rotating path where the centrifugal force is the weakest, such a platelet path forms a boundary line between on the one hand, the platelet-poor plasma (PPP) on the inner side of said path and on the other hand, the platelet-rich plasma (PRP) on the outer side of said path.

The problem underlying the present invention is to provide a method and device according to which it is possible to substantially reduce the length of the platelet paths, to correspondingly increase the sedimentation speed as well as to improve the platelet recovery efficency.

The method according to the invention is characterized by continuously collecting the platelet-poor plasma as it is formed, along at least a portion of the length of said flow.

According to the invention, the method is carried out in a separating device of the above-mentionned type wherein at least a portion of the length of said inner circular wall, one end of which is adjacent to said inlet opening, comprises at least one opening adapted to be connected to suction means.

The present invention will be explained hereafter with reference to the drawings wherein:
Figure 1 is a platelet path diagram in a flow of plasma submitted to centrifugal force;
Figure 2 is a platelet path diagram in a flow of plasma submitted to a centrifugal force with suction of plasma on the inner side of the platelet path;
Figure 3 is a plan view of an embodiment for carrying out the method according to the invention;
Figure 4 is a sectional view according to line IV-IV of figure 3;
Figure 5 is a plan view of another embodiment for carrying out this method.

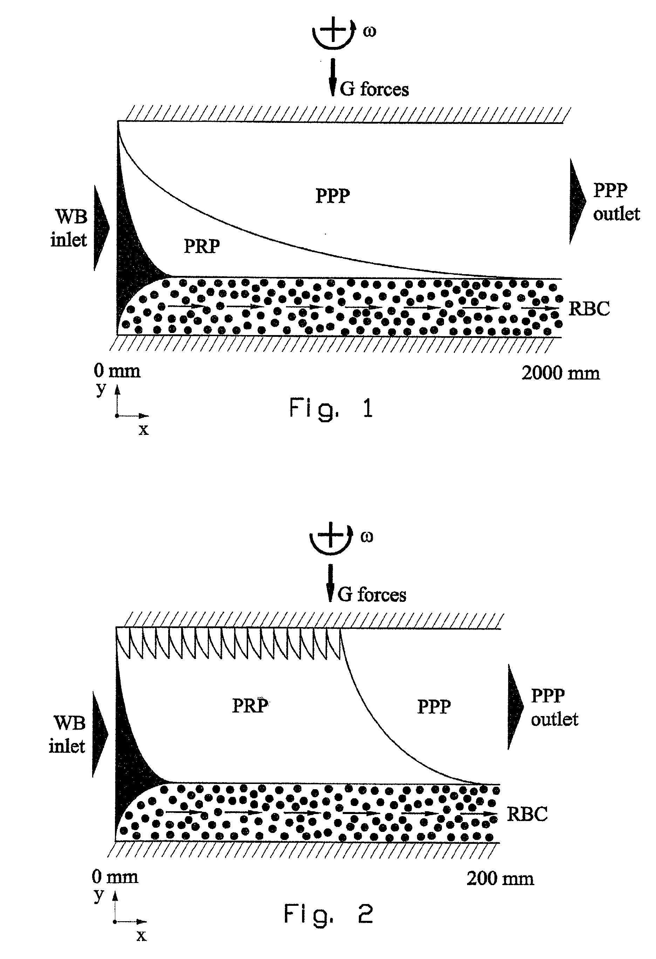

In the diagram of figure 1, the thickness of the separating room, that is to say the distance between the inner and outer walls of this room, which is transversal to the flow direction of blood to be separated, corresponds to the y co-ordinate, whereas the x co-ordonate corresponds to the flow direction of this blood between upstream and downstream ends of this separating room. This room has preferably a circular shape and is rotated around its central axis so that the blood flow through this room is simultaneously submitted to a centrifugal force field. Due to the higher density of the red blood cells (RBC) they deposit at first against the external wall of the upstream section of the separating room where they are collected.

However, the x dimension of the separating room is set by the length of the platelet sedimentation path which is 15 times longer than that of red blood cells. Since the flow in the separation room is substantially laminar, and since in that case the outer wall is actually formed by the red blood cell flowing, the speed profil is substantially triangular, so that the platelet sedimentation path has the curve profil illustrated in figure 1.

It may be observed that "above" or inside the curve of platelet sedimentation path in figure 1 (with respect to the center of rotation when the separating room has a circular shape), in the flow part situated in the smallest radius of the separation room, there is only platelet-poor plasma PPP, whereas "under" or outside this curve, in the flow part situated in the larger radius of this room, there is only platelet-rich plasma PRP to be concentrated into platelet concentrate (PC), which is the blood component to be recovered in this case.

If PPP is collected as it is formed, for instance by connecting the inner side of a circular separating room to a suction source, or by feeding the whole blood under pressure, as it will be explained hereunder, three effects result from this PPP collection:
- The boundary line between PRP and PPP moves towards the inner side of the circular separating room, as it may be observed on the diagram of figure 2. This first effect could be detrimental if the suction flow rate were not well controlled, so that an unwanted part of PRP could be collected simultaneously with PPP.
- Due to the suction of PPP or its driving out of the separation room, the overall flow rate decreases so that the slope of the platelet sedimentation path sharply increases downstream from the suction, reducing substantially the sedimentation time as well as the length of sedimentation path.
- Since following the suction of PPP or its driving out of the separation room, the overall flow rate decreases, the separation between PPP and PRP is made easier.

The PPP has to be sucked or driven out at the very beginning of the separation between PPP and PRP, where the slope of the platelet sedimentation path is the sharpest, to be effective. If the suction source is connected to the inner side of the separation room by a plurality of openings, as illustrated in the diagram of figure 2, the length of the separation room may dramatically decrease from 2 meters to 200 mm.

According to the diagrams of figures 1 and 2, the platelet sedimentation path in a whole blood flowing into a circular room rotating around its central axis corresponds to x and y speeds at once. If one takes into consideration the path of the smallest platelet starting at the innermost radius of the separation room, that is to say the less favorable sedimentation condition, as illustrated in figure 2, this path forms also the boundary line which separates PPP and PRP. Therefore, if the length of the separation room is at least as long as that of the above boundary line according to the x co-ordinate, the plasma collected at an outlet opening of the separation room is substantially free from platelets.

It may be observed from the above statement that the separation method according to the invention is followed by a very important reduction of sedimentation time and by that of the length of the sedimentation path and therefore by a reduction of the overall dimensions of the separation member. Such a smaller separation member is particularly adapted to be produced as a disposable member and gives at the same time the posssibility to reduce the dimensions of centrifugal apparatus. Moreover this method is followed by a substantial improvement of the platelet collection efficency.

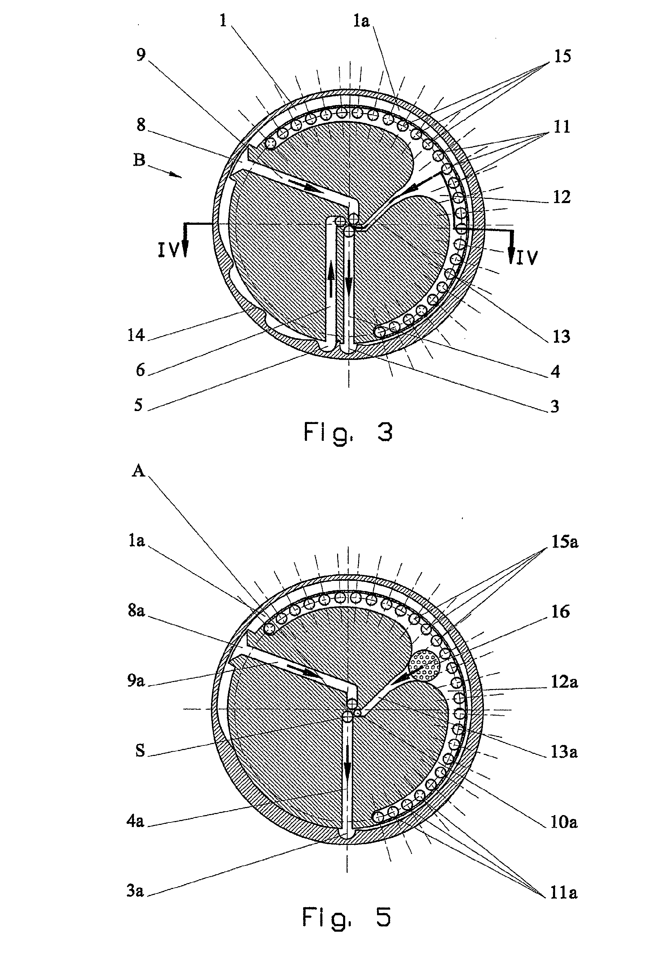

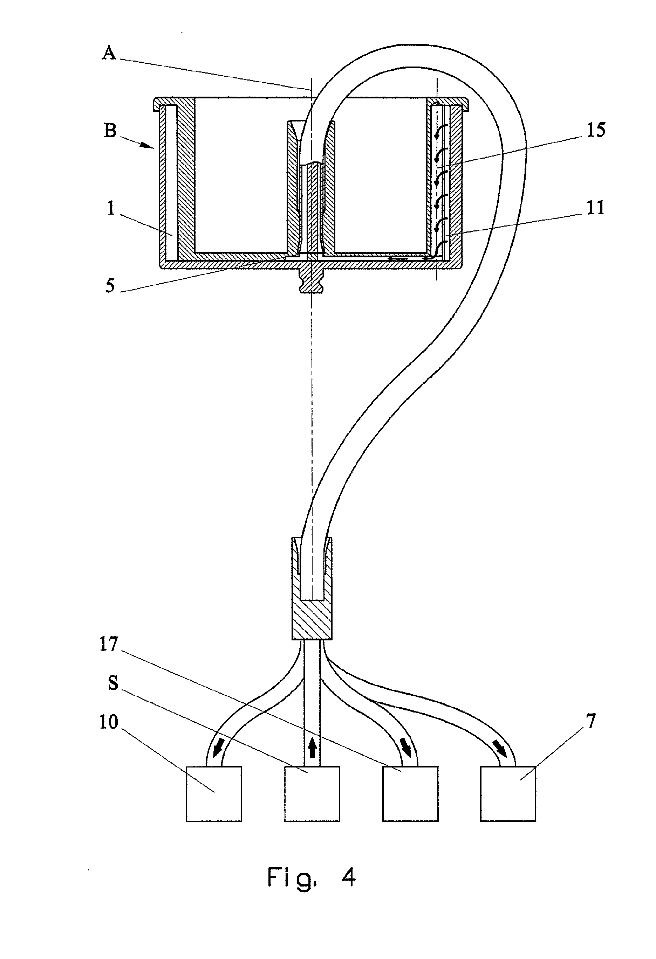

The separation device for carrying out this separation method, depicted in Figures 3 and 4 comprises a separation room 1 which is circularly shaped around a central axis A of a centrifugal bowl B. This room 1 has a generally narrow rectangular cross-sectional form, the long sides of which are parallel to the central axis A, so that a centrifugal force field results from the rotation of the separation room 1 around the central axis A.

The upstream end of the separation room 1 with respect to the blood flow direction, has an inlet opening 3 connected to a supply S of whole blood (WB) by a feeding tube 4, and its downstream end has an outlet opening 5 connected by a discharge tube 6 into a platelet concentrate (PC) collector 7. In this case, the supply S is preferably formed by a peristaltic pump so that the pressure in the separation room 1 is higher than the atmospheric pressure. Another outlet opening 8 is at an end of a first separating section 1a of the room 1, in which substantially all deposited RBC are gathered along the outer wall of the room 1. This other outlet opening 8 is situated between the inlet opening 3 and the downstream end outlet opening 5 and is provided at an end of a discharge tube 9 adjacent to the outer wall of the separation room 1 with respect to the central axis A. This discharge tube 9 is connected to a collector container 10 which is at the atmospheric pressure so that since the pressure inside the separation room 1 is higher than the atmospheric pressure, the RBC gathered along the outer wall of the separation room 1 are driven out through the outlet opening 8.

A collector channel 12 is connected on the one hand, to a collector container 17 through a discharge tube 13, and on the other hand to the first separating section of the separation room 1 through a plurality of openings 11 provided through the inner wall of said first separating section of the separation room 1 with respect to the central axis A. All these openings 11 are connected to the collector channel 12, through separate divider elements 15 for evenly distributing the relative depressure between the collector channel 12 and the separation room 1. The PPP driven out the separation room 1 through the discharge tube 13 and collected into the collector container 17 will be thereafter re-mixed with RBC to be continuously returned to the donor.

Preferably, as depicted in figure 4, the openings 11 have the form of elongated slits extending substantially on the overall height of the separation room 1 and the divider elements 15 have a tubular form. Similarly, the inlet opening 3 and the outlet openings 5 and 8 extend also in a parallel direction to the central axis, on the overall height of the separation room.

Downstream to the first separating section of the room 1, the outer wall of this room 1 comprises leucocyte trapping recesses 14 arranged in series which cause the leucocyte to separate from the platelet concentrate (PC) which is collected through the discharge tube 6 into the PC collector 7.

According to an example of the separating method of the invention, the WB flow rate was 60 ml/min which was separated into 18 ml/min of PPP, 34 ml/min of RBC and 8 ml of PC. In this example the suction of PPP was distributed between 27 slits parallel to the central axis A of the centrifugal bowl B.

According to the specific case, the WB flow rate may be increased up to around 300 ml/min and the ratio between the flow rates of PPP, RBC and PC may be adapted in relation with the required PC concentration.

The embodiment of figure 5 differs from that of figures 3 and 4 principally by the component to be collected. Instead of collecting platelets this embodiment relates to a plasmapheresis method. Indeed, taking into account the enhancing platelet recovery efficency of the separation method according to this invention, the collected plasma is substantially free from platelets, so that this process would be also well suited for extraction of substantially free platelet plasma from whole blood. In this embodiment, the separation room 1a comprises only one inlet opening 3a connected to a supply S of whole blood (WB) by a feeding tube 4a and one outlet opening 8a provided at the end of a discharge tube 9a. A plurality of openings 11a are provided through the inner wall of the separation room 1a with respect to the central axis A. All these openings 11a are connected to a same collector channel 12a, through separate divider elements 15a for evenly distributing the depressure of the collector channel 12a which is connected to a suction source 10a through a discharge tube 13a. Preferably, a filtering member 16 is placed through the inlet end of this discharge tube 13a in order to prevent any possible platelet to be collected with the plasma.

## Claims

1. Method of continuously separating whole blood as well as any liquid containing human cells comprising the steps of: forming a substantially laminar flow of said blood and submitting said flow to a force field directed transversely to the direction of said flow to get the red blood cells and at least the platelets separated, and discharging said separated components out of said flow, **characterized by** continuously collecting the platelet-poor plasma as it is formed, along at least a portion of the length of said flow.

2. Method according to claim 1 wherein said blood flow is formed along a circular path which is rotated around its central axis to submit said flow to a centrifugal force field, said platelet-poor plasma being collected along at least a portion of the inner side of said circular path.

3. Method according to claim 2, wherein said portion of the inner side of said circular path along which the platelet-poor plasma is collected is connected to suction means.

4. Method according to claim 3, wherein the suction of said source is splitted up into a plurality of places of the inner side of said circular path.

5. Method according to one of claims 2 to 4, wherein the length of said portion of the inner side of said circular path is sized for collecting substantially said platelet-poor plasma as a whole.

6. Method according to one of claims 2 to 5, wherein the red blood cells are collected near the outer side of said circular path, downstream from said portion of the inner side of said circular path along which said platelet-poor plasma is collected.

7. A continuous separating device for whole blood as well as any liquid containing human cells which comprises a circular centrifuge member adapted for rotation about its central axis in a centrifuge, said member having two outer, respectively inner circular walls spaced from each other to define a separating room comprising an inlet opening to be connected to a supply of blood and at least one outlet opening to be connected to means for collecting a higher density component of blood, said inlet and outlet openings being respectively adjacent to upstream, respectively downstream ends angularly spaced of said separating room, **characterized in that** at least a portion of the length of said inner circular wall, one end of which is adjacent to said inlet opening comprises at least one opening adapted to be connected to suction means.

8. The separating device according to claim 7, wherein said separating room comprises another outlet opening to be connected to means for collecting a lower density component, which is situated downstream from said outlet opening for collecting a higher density component of blood.

9. The separating device according to one of claims 7 and 8, wherein the cross-section of said separating room has a generally narrow rectangular cross-sectional form, the long sides of which are parallel to said central axis, a plurality of slits parallel to said central axis being provided through said inner circular wall.

10. The separating device according to one of claim 7 and 8, wherein said separating room comprises two sections, a first section for separating and collecting RBC and PPP and a second section comprising leucocyte trapping recesses arranged in series.

11. The separating device according to claim 9, wherein each of said slits open into a divider element, all said divider elements being connected to a suction source through a collector channel.

12. The separating device according to claim 9, wherein said outlet opening for PPP collection and said other outlet opening for RBC collection are connected to a same suction source.
